(19) Europäisches Patentamt
European Patent Office
Office européen des brevets

(11) Veröffentlichungsnummer: **0 422 450 A2**

(12) **EUROPÄISCHE PATENTANMELDUNG**

(21) Anmeldenummer: **90118543.9**

(22) Anmeldetag: **27.09.90**

(51) Int. Cl.5: **C07D 407/12**, C07D 409/12, C07D 311/58, C07D 311/68, C07D 311/70, C07D 311/96, A61K 31/35

(30) Priorität: **09.10.89 DE 3933663**

(43) Veröffentlichungstag der Anmeldung: **17.04.91 Patentblatt 91/16**

(84) Benannte Vertragsstaaten: **AT BE CH DE DK ES FR GB GR IT LI LU NL SE**

(71) Anmelder: **MERCK PATENT GESELLSCHAFT MIT BESCHRÄNKTER HAFTUNG**
**Frankfurter Strasse 250 Postfach 4119**
**W-6100 Darmstadt(DE)**

(72) Erfinder: **Gericke, Rolf, Dr.**
**Mozartstrasse 19**
**W-6104 Seeheim(DE)**
Erfinder: **Baumgarth, Manfred, Dr.**
**Sachsenstrasse 53**
**W-6100 Darmstadt(DE)**
Erfinder: **Lues, Ingeborg, Dr.**
**Paul-Wagner-Strasse 13**
**W-6100 Darmstadt(DE)**
Erfinder: **De Peyer, Jacques, Dr.**
**Weisenbühlweg 33K**
**CH-3007 Bern(CH)**
Erfinder: **Bergmann, Rolf, Dr.**
**Birkenhag 36**
**W-6101 Reichelsheim(DE)**

(54) **Chromanderivate.**

(57) Neue Chromderivate der Formel I

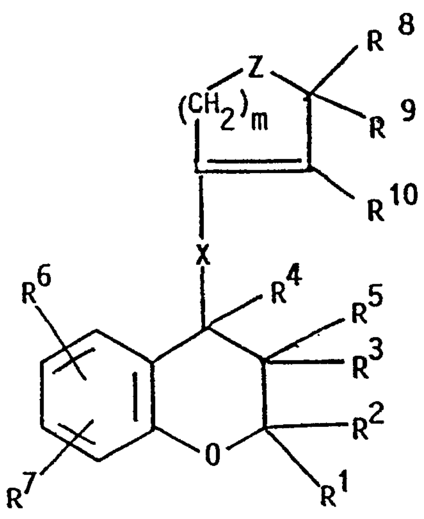

I

worin X, Z, $R^1$ bis $R^{10}$ und m die in Patentanspruch 1 angegebenen Bedeutungen haben, sowie ihre Salze zeigen Wirkungen auf das cardiovaskuläre System.

EP 0 422 450 A2

# CHROMANDERIVATE

Die Erfindung betrifft neue Chromanderivate der Formel I

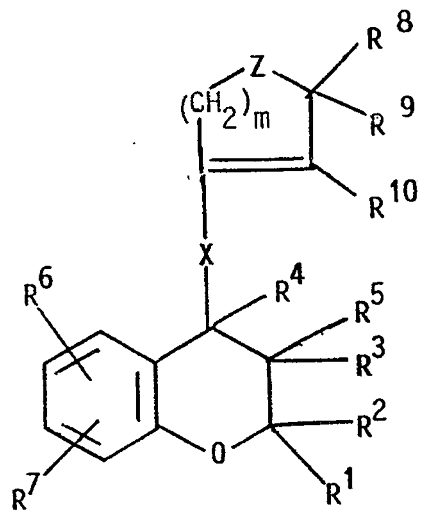

worin

X O oder $NR^{11}$,

Z $CH_2$, O, S oder CHHal,

$R^1$ und $R^5$ jeweils A,

$R^2$ H oder A,

$R^1$ und $R^2$ zusammen auch Alkylen mit 3-6 C-Atomen,

$R^3$ OH oder OAc,

$R^4$ H,

$R^3$ und $R^4$ zusammen auch eine Bindung,

$R^6$ und $R^7$ jeweils H, A, HO, AO, CHO, ACO, ACS, HOOC, AOOC, AO-CS, ACOO, A-CS-O, Hydroxyalkyl, $NO_2$, $NH_2$, NHA, $NA_2$, CN, F, Cl, Br, J, $CF_3$, ASO, $ASO_2$, AO-SO, AO-$SO_2$, AcNH, AO-CO-NH, $H_2NSO$, HANSO, $A_2NSO$, $H_2NSO_2$, $HANSO_2$, $A_2NSO_2$, $H_2NCO$, HANCO, $A_2NCO$, $H_2NCS$, HANCS, $A_2NCS$, ASONH, $ASO_2NH$, AOSONH, $AOSO_2NH$, ACO-alkyl, Nitro-alkyl, Cyan-alkyl, A-C(=NOH) oder A-C(=$NNH_2$),

$R^8$ und $R^9$ jeweils H oder A oder zusammen =O oder =S,

$R^{10}$ H, Hal, CHO oder $CH_2OH$,

$R^{11}$ H, A, Ac oder $CH_2OH$,

m 1, 2 oder 3,

Hal F, Cl, Br oder J,

A Alkyl mit 1-6 C-Atomen,

alkyl Alkylen mit 1-6 C-Atomen und

Ac Alkanoyl mit 1-8 C-Atomen oder Aroyl mit 7-11 C-Atomen

bedeuten,

sowie deren Salze.

Ähnliche Verbindungen sind bekannt aus der GB-A-2204868.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze bei guter Verträglichkeit wertvolle pharmakologische Eigenschaften besitzen. So zeigen sie Wirkungen auf das cardiovaskuläre System, wobei in der Regel bei niedrigeren Dosen ein bevorzugter Angriff am Coronarsystem und am Bronchialsystem, bei höheren ein zusätzlicher blutdrucksenkender Effekt beobachtet werden kann. Am Coronarsystem treten z. B. Widerstandsabnahme und Flußzunahme auf, wobei der Einfluß auf die Herzfrequenz gering bleibt. Weiterhin zeigen die Verbindungen eine relaxierende Wirkung auf verschiedene glattmuskuläre Organe (Gastrointestinaltrakt, Respirationssystem und Uterus). Die Wirkungen der Verbindungen können mit Hilfe an sich bekannter Methoden ermittelt werden, wie sie z. B. in der EP-A1-76075, der EP-A1-173848 oder der AU-A-45547/85 (Derwent Farmdoc Nr. 86081769) sowie von K.S. Meesmann et al., Arzneimittelforschung 25 (11), 1975, 1770-1776, angegeben sind. Als Versuchstiere eignen sich z. B. Mäuse, Ratten, Meerschweinchen, Hunde, Katzen, Affen oder Schweine.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

In den angegebenen Formeln bedeutet A eine vorzugsweise unverzweigte Alkylgruppe mit 1-6, bevorzugt 1-4, insbesondere 1, 2 oder 3 C-Atomen, im einzelnen vorzugsweise Methyl, ferner bevorzugt Ethyl, Propyl, Isopropyl, Butyl, Isobutyl, weiterhin bevorzugt sek.-Butyl, tert.-Butyl, Pentyl, Isopentyl (3-Methylbutyl), Hexyl oder Isohexyl (4-Methylpentyl).

Falls $R^1$ und $R^2$ zusammen Alkylen bedeuten, so ist die Alkylengruppe vorzugsweise unverzweigt, im einzelnen bevorzugt $-(CH_2)_n-$, wobei n 3, 4, 5 oder 6 bedeutet.

Die Gruppe "-alkyl" steht vorzugsweise für $-CH_2-$ oder $-CH_2CH_2-$.

Ac ist vorzugsweise Alkanoyl mit 1-6, insbesondere 1 ,2, 3 oder 4 C-Atomen, im einzelnen bevorzugt Formyl oder Acetyl, weiterhin bevorzugt Propionyl, Butyryl, Isobutyryl, Pentanoyl oder Hexanoyl, ferner bevorzugt Benzoyl, o-, m- oder p-Toluyl, 1- oder 2-Naphthoyl.

X ist vorzugsweise O, ferner bevorzugt NH.

Z ist vorzugsweise $CH_2$, ferner bevorzugt O.

$R^1$ und $R^2$ sind vorzugsweise jeweils Alkyl, insbesondere jeweils Methyl oder Ethyl, bevorzugt jeweils Methyl.

Falls $R^4$ H bedeutet, ist $R^3$ bevorzugt OH, ferner O-CHO oder $O-COCH_3$.

$R^5$ ist bevorzugt Methyl, ferner Ethyl.

In $R^6$ und $R^7$ bedeuten vorzugsweise:

A: Methyl, ferner Ethyl;
AO: Methoxy, ferner Ethoxy;
ACO: Acetyl, ferner Propionyl;
ACS: Thioacetyl, ferner Thiopropionyl;
AOOC: Methoxycarbonyl, ferner Ethoxycarbonyl;
AO-CS: Methoxy-thiocarbonyl, ferner Ethoxy-thiocarbonyl;
ACOO: Acetoxy, ferner Propionoxy;
ACSO: Thio(no)acetoxy, ferner Thio(no)propionoxy;
Hydroxyalkyl: Hydroxymethyl oder 1- oder 2-Hydroxyethyl;
Mercaptoalkyl: Mercaptomethyl oder 1- oder 2-Mercaptoethyl;
NHA: Methylamino, ferner Ethylamino;
$NA_2$: Dimethylamino, ferner Diethylamino;
ASO: Methylsulfinyl, ferner Ethylsulfinyl;
$ASO_2$: Methylsulfonyl, ferner Ethylsulfonyl;
AO-SO: Methoxy-sulfinyl, ferner Ethoxy-sulfinyl;
$AO-SO_2$: Methoxy-sulfonyl, ferner Ethoxy-sulfonyl;
Ac-NH: Acetamido, ferner Formamido, Propionamido oder Benzamido;
AO-CO-NH: Methoxycarbonylamino, ferner Ethoxycarbonylamino;
HANSO: Methylaminosulfinyl, ferner Ethylaminosulfinyl
$A_2NSO$: Dimethylaminosulfinyl, ferner Diethylaminosulfinyl;
$HANSO_2$: Methylaminosulfonyl, ferner Ethylaminosulfonyl;
$A_2NSO_2$: Dimethylaminosulfonyl, ferner Diethylaminosulfonyl;
HANCO: N-Methylcarbamoyl, ferner N-Ethylcarbamoyl;
$A_2NOC$: N,N-Dimethylcarbamoyl, ferner N,N-Diethylcarbamoyl;
HANCS: N-Methyl-thiocarbamoyl, ferner N-Ethylthiocarbamoyl;
$A_2NCS$: N,N-Dimethyl-thiocarbamoyl, ferner N,N-Diethyl-thiocarbamoyl;
ASONH: Methylsulfinylamino, ferner Ethylsulfinylamino;
$ASO_2NH$: Methylsulfonylamino, ferner Ethylsulfonylamino;
AOSONH: Methoxysulfinylamino, ferner Ethoxysulfinylamino;
$AOSO_2NH$: Methoxysulfonylamino, ferner Ethoxysulfonylamino;
ACO-alkyl: 2-Oxopropyl, 2-Oxobutyl, 3-Oxobutyl, 3-Oxopentyl;
Nitroalkyl: Nitromethyl, 1- oder 2-Nitroethyl;
Cyanalkyl: Cyanmethyl, 1- oder 2-Cyanethyl;
A-C(= NOH): 1-Oximinoethyl, ferner 1-Oximinopropyl;

A-C(= $NNH_2$): 1-Hydrazonoethyl, ferner 1-Hydrazonopropyl.

Die Reste $R^6$ und $R^7$ stehen vorzugsweise in 6- und 7-Stellung des Chromansystems. Sie können jedoch auch in 5- und 6-, 5- und 7-, 5- und 8-, 6- und 8- sowie 7- und 8-Stellung stehen.

Von den Resten $R^6$ und $R^7$ ist vorzugsweise der eine H, während der andere von H verschieden ist. Dieser andere Rest steht vorzugsweise in 6-Stellung, aber auch in 5-, 7- oder 8-Stellung, und ist vorzugsweise CN oder $NO_2$, ferner bevorzugt CHO, ACO (insbesondere Acetyl), AOOC (insbesondere Methoxycarbonyl oder Ethoxycarbonyl), ACOO (insbesondere Acetoxy), weiterhin bevorzugt F, Cl, Br, J, $CF_3$, $H_2NCO$, $H_2NCS$ oder $NH_2$.

$R^8$ und R9 sind vorzugsweise zusammen =O.

$R^{10}$ und $R^{12}$ sind vorzugsweise jeweils H.

$R^{11}$ ist vorzugsweise H.

Der Parameter m ist vorzugsweise 1, ferner bevorzugt 2.

Hal ist vorzugsweise Cl oder Br.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die nachstehenden Formeln Ia bis Ie ausgedrückt werden, die der Formel I entsprechen und worin die nicht näher bezeichneten Reste die bei der Formel I angegebene Bedeutung haben, worin jedoch

in Ia $R^1$ und $R^2$ jeweils A bedeuten;

in Ib $R^1$ und $R^2$ jeweils $CH_3$ bedeuten;

in Ic $R^1$ und $R^2$ zusammen Alkylen mit 3-6 C-Atomen bedeuten;

in Id $R^5$ $CH_3$ bedeutet;

in Ig $R^1$, $R^2$ und $R^5$ jeweils $CH_3$ bedeuten.

Weiterhin sind bevorzugt Verbindungen der Formeln I′ sowie Ia′ bis Ie′, die den Formeln I sowie Ia bis Ie entsprechen, worin jedoch jeweils zusätzlich $R^3$ OH und $R^4$ H bedeuten.

Weiterhin sind bevorzugt Verbindungen der Formeln I″ sowie Ia″ bis Ie″, die den Formeln I sowie Ia bis Ie entsprechen, worin jedoch jeweils zusätzlich

X O,

Z $CH_2$,

m 1,

$R^8$ und $R^9$ zusammen =O und

$R^{10}$ H bedeuten.

Ferner sind bevorzugt Verbindungen der Formeln I, I′, I″, Ia bis Ie, Ia′ bis Ie′ sowie Ia″ bis Ie″, worin jeweils zusätzlich

(a) $R^6$ von H verschieden ist und
$R^7$ H bedeutet;

(b) $R^6$ von H verschieden ist und in 6-Stellung steht und
$R^7$ H bedeutet;

(c) $R^6$ $NO_2$, CN, CHO, ACO, HOOC, AOOC, ACOO, F, Cl, Br, J, $CF_3$, $H_2NCO$, $H_2NCS$ oder $NH_2$ und
$R^7$ H bedeutet;

(d) $R^6$ $NO_2$, CN, CHO, ACO, HOOC, AOOC, ACOO, F, Cl, Br, J, CF $H_2NCO$, $H_2NCS$ oder $NH_2$ bedeutet und in 6-Stellung steht und
$R^7$ H bedeutet;

(e) $R^6$ $NO_2$, CN, CHO, $CH_3CO$, $CH_3OOC$,$C_2H_5OOC$ oder $CH_3COO$ und
$R^7$ H bedeutet;

(f) $R^6$ $NO_2$, CN, CHO, $CH_3CO$, $CH_3OOC$,$C_2H_5OOC$ oder $CH_3COO$ bedeutet und in 6-Stellung steht und
$R^7$ H bedeutet;

(g) $R^6$ $NO_2$ oder CN und
$R^7$ H bedeutet;

(h) $R^6$ $NO_2$ oder CN bedeutet und in 6-Stellung steht und
$R^7$ H bedeutet;

(i) $R^6$ CN und
$R^7$ H bedeutet;

(j) $R^6$ CN bedeutet und in 6-Stellung steht und
$R^7$ H bedeutet.

Im übrigen haben vor- und nachstehend die Reste bzw. Parameter X, Z, $R^1$ bis $R^{11}$, m, Hal, A, "-alkyl" und Ac die bei Formel I angegebenen Bedeutungen, wenn nicht ausdrücklich etwas anderes angegeben ist.

Gegenstand der Erfindung ist ferner ein Verfahren zur Herstellung von Chromanderivaten der Formel I, dadurch gekennzeichent, daß man ein 3,4-Epoxychroman der Formel II

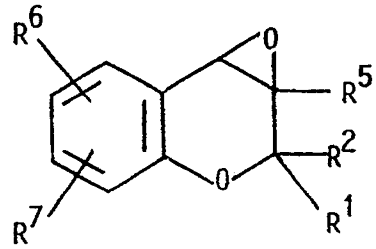

II

worin
$R^1$, $R^2$, $R^5$, $R^6$ und $R^7$ die bei Formel I angegebene Bedeutung haben,
mit einer Verbindung der Formel III

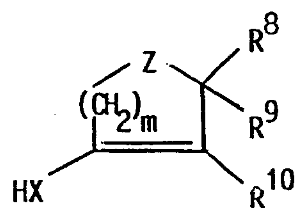

III

worin X, Z, $R^8$, $R^9$, $R^{10}$ und m die bei Formel I angegebene Bedeutung haben oder mit einem ihrer reaktionsfähigen Derivate umsetzt
und/oder daß man eine Verbindung der Formel I, worin $R^3$ OH und $R^4$ H bedeutet, dehydratisiert und/oder daß man in einer Verbindung der Formel I einen oder mehrere der Reste X, Z, $R^3$, $R^6$ und /oder $R^7$ in andere Reste X, Z, $R^3$, $R^6$ und/oder $R^7$ umwandelt und/oder daß man eine basische Verbindung der Formel I durch Behandeln mit einer Säure in eines ihrer Säureadditionssalze umwandelt.

Die Verbindungen der Formel I werden im übrigen nach an sich bekannten Methoden hergestellt, wie sie in der Literatur (z. B. in den Standardwerken wie Houben-Weyl, Methoden der organischen Chemie, Georg-Thieme-Verlag, Stuttgart; Organic Reactions, John Wiley & Sons, Inc., New York; sowie in den oben angegebenen Patentanmeldungen) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Vorzugsweise werden die Verbindungen der Formel I durch Reaktion von Verbindungen der Formel II mit Verbindungen der Formel III hergestellt, zweckmäßig in Gegenwart eines inerten Lösungsmittels bei Temperaturen zwischen etwa 0 und 150 °, vorzugsweise 15 und 30 °.

Die Ausgangsstoffe II und III sind in der Regel bekannt. Sofern sie nicht bekannt sind, können sie nach an sich bekannten Methoden hergestellt werden. Die Ausgangsstoffe der Formel II sind erhältlich durch Umsetzung von 2-Hydroxyacetophenonen der Formel 2-HO-$R^6R^7C_6H_2$-$COCH_3$ mit Ketonen der Formel $R^1$-CO-$R^2$ zu entsprechenden 4-Chromanonen der Formel IVa,

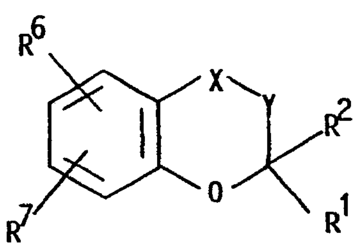

IVa -X-Y- = $-CO-CH_2-$
IVb -X-Y- = $-CO-C(=CH-R^{12})-$
IVc -X-Y- = $-CHOH-CHR^5-$
IVd -X-Y- = $-CH=CR^5-$
IVe -X-Y- = $-CHBr-CR^5OH-$

Kondensation mit Aldehyden der Formel $R^{12}$-CHO ($R^{12}$ = Alkyl mit 1-5 C-Atomen) zu 3-Alkyliden-4-chromanonen der Formel IVb,Reduktion, z.B. mit $NaBH_4$ zu 3-Alkyl-4-chromanolen der Formel IVc Dehydratisierung, z.B. mit p-Toluolsulfonsäure, zu Chromenen der Formel IVd und Oxydation, z.B. mit 3-Chlorperbenzoesäure. Die letztgenannte Oxydation kann auch mehrstufig erfolgen. So kann man, z.B. mit N-Bromsuccinimid in wäßriger Lösung, zunächst die Bromhydrine der Formel IVe herstellen und diese anschließend mit einer Base, z.B. Natronlauge, behandeln.

Man kann die Chromene der Formel IVd auch erhalten durch Kondensation von Salicylaldehyden der Formel 2-HO-$R^6R^7C_6H_2$-CHO mit Ketonen der Formel $R^1$-CO-$CH_2$-$R^5$ zu Hydroxyketonen der Formel 2-HO-$R^6R^7C_6H_2$-CH = $CR^5$-CO-$R^1$, Umsetzung mit Organo-Li-Verbindungen der Formel $R^2$-Li und nachfolgende Hydrolyse zu Diolen der Formel 2-HO-$R^6R^7C_6H_2$-CH = $CR^5$-$CR^1R^2$-OH und Cyclisierung unter Wasserabspaltung.

Als reaktionsfähige Derivate von III eignen sich die entsprechenden Salze, z. B. die Na- oder K-Salze, die auch in situ entstehen können.

Es ist zweckmäßig, in Gegenwart einer Base zu arbeiten. Als Basen eignen sich z. B. Alkalimetall- oder Erdalkalimetall-hydroxide, -hydride oder auch -amide wie NaOH, KOH, $Ca(OH)_2$, NaH, KH, $CaH_2$, $NaNH_2$, $KNH_2$, ferner organische Basen wie Triethylamin oder Pyridin, die auch im Überschuß angewendet werden können und dann gleichzeitig als Lösungsmittel dienen. Oft ist es ferner zweckmäßig, katalytische oder stöchiometrische Mengen von $Cu_2Br_2$, $MgBr_2$, Titan-alkoxiden oder Lewissäuren wie $BF_3$-Etherat zuzusetzen.

Als inerte Lösungmittel eignen sich insbesondere Alkohole wie Methanol, Ethanol, Isopropanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Nitrile wie Acetonitril; Nitroverbindungen wie Nitromethan oder Nitrobenzol; Ester wie Ethylacetat; Amide wie Dimethylformamid (DMF), Dimethylacetamid oder Phosphorsäurehexamethyltriamid; Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid; Kohlenwasserstoffe wie Benzol, Toluol oder Xylol. Weiterhin eignen sich Gemische dieser Lösungsmittel untereinander.

Das Epoxid II kann auch in situ hergestellt werden, z. B. durch Einwirkung einer Base auf das entsprechende Bromhydrin Ve.

Eine Verbindung der Formel I, worin $R^3$ = OH und $R^4$ = H ist, kann durch Behandeln mit einem Dehydratisierungsmittel in eine Verbindung der Formel I, worin $R^3$ und $R^4$ zusammen eine Bindung bedeuten, umgewandelt werden. Das gelingt z. B. durch Einwirkung einer der angegebenen Basen, z. B. NaOH, KOH oder NaH, in einem der angegebenen Lösungsmittel, z. B. Tetrahydrofuran, Dioxan oder DMSO, bei Temperaturen zwischen 0 und 150°.

Weiterhin kann man in einer Verbindung der Formel I einen oder mehrere der Reste X, Z, $R^3$, $R^6$ und/oder $R^7$ in andere Reste X, Z, $R^3$, $R^6$ und/oder $R^7$ umwandeln.

Beispielsweise ist es möglich, daß man ein H-Atom mittels einer Halogenierung durch ein Halogenatom oder einer Nitrierung durch eine Nitrogruppe ersetzt und/oder eine Nitrogruppe zu einer Aminogruppe reduziert und/oder eine Amino- oder Hydroxygruppe alkyliert oder acyliert und/oder eine Cyangruppe (z.B. mit HCl in Wasser/Methanol bei 20-100°) in eine Carboxylgruppe oder (z. B. mit Raney-Nickel in Wasser/Essigsäure/Pyridin in Gegenwart von Natriumphosphat) in eine Formylgruppe oder (z. B. mit KOH in tert.-Butanol) in eine Carbamoylgruppe oder (z. B. mit $H_2S$ in Pyridin/Triethylamin) in eine Thiocarbamoylgruppe umwandelt.

Eine Nitrierung gelingt unter üblichen Bedingungen, z. B. mit einem Gemisch aus konzentrierter $HNO_3$ und konzentrierter $H_2SO_4$ bei Temperaturen zwischen 0 und 30°.

Eine Halogenierung kann z. B. mit elementarem Chlor oder Brom in einem der üblichen inerten Lösungsmittel bei Temperaturen zwischen etwa 0 und 30 ° durchgeführt werden.

Eine primäre oder sekundäre Aminogruppe und/oder eine OH-Gruppe kann durch Behandeln mit alkylierenden Mitteln in die entsprechende sekundäre oder tertiäre Aminogruppe und/oder Alkoxygruppe umgewandelt werden. Als alkylierende Mittel eignen sich z. B. Verbindungen der Formeln A-Cl, A-Br oder A-J oder entsprechende Schwefelsäure- oder Sulfonsäureester wie Methylchlorid, -bromid, -jodid, Dimethylsulfat, Methyl-p-toluolsulfonat. Ferner kann man z. B. eine oder zwei Methylgruppen mit Formaldehyd in Gegenwart von Ameisensäure einführen. Die Alkylierung wird zweckmäßig in Gegenwart oder Abwesenheit eines der genannten inerten Lösungemittel, z. B. DMF, bei Temperaturen zwischen etwa 0 ° und etwa 120 ° vorgenommen, wobei auch ein Katalysator zugegen sein kann, vorzugsweise eine Base wie Kalium-tert.-butylat oder NaH.

Als acylierende Mittel zur Acylierung von Amino- oder Hydroxygruppen eignen sich zweckmäßig die

Halogenide (z. B. Chloride oder Bromide) oder Anhydride von Carbonsäuren der Formel Ac-OH, z. B. Acetanhydrid, Propionylchlorid, Isobutyrylbromid, Ameisensäure/Essigsäureanhydrid, Benzoylchlorid. Der Zusatz einer Base wie Pyridin oder Triethylamin bei der Acylierung ist möglich. Man acyliert zweckmäßig in Gegenwart oder Abwesenheit eines inerten Lösungsmittels, z. B. eines Kohlenwasserstoffs wie Toluol, eines Nitril wie Acetonitril, eines Amids wie DMF oder eines Überschusses einer tertiären Base wie Pyridin oder Triethylamin bei Temperaturen zwischen etwa 0 ° und etwa 160 °, vorzugsweise zwischen 20 ° und 120 °. Eine Formylierung gelingt auch mit Ameisensäure in Gegenwart von Pyridin.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und -disulfonsäuren, Laurylschwefelsäure. Salze mit physiologisch nicht unbedenklichen Säuren, z. B. Pikrate, können zur Aufreinigung der Verbindungen der Formel I verwendet werden.

Die Verbindungen der Formel I können ein oder mehrere chirale Zentren besitzen..Sie können daher bei ihrer Herstellung als Racemate oder, falls optisch aktive Ausgangsstoffe verwendet werden, auch in optisch aktiver Form erhalten werden. Weisen die Verbindungen zwei oder mehr chirale Zentren auf, dann können sie bei der Synthese als Gemische von Racematen anfallen, aus denen man die einzelnen Racemate, beispielsweise durch Umkristallisieren aus inerten Lösungsmitteln, in reiner Form isolieren kann. So haben z. B. Verbindungen der Formel I, worin $R^1$ = $R^2$, $R^3$ = OH und $R^4$ = H ist, zwei chirale Zentren; bei der Herstellung durch Reaktion von II mit III entsteht jedoch ganz überwiegend nur ein Racemat mit trans-Stellung der Substituenten $R^3$ = OH und $R^5$. Erhaltene Racemate können, falls erwünscht, nach an sich bekannten Methoden mechanisch oder chemisch in ihre Enantiomeren getrennt werden. So können aus dem Racemat durch Umsetzung mit einem optisch aktiven Trennmittel Diastereomere gebildet werden. Als Trennmittel für basische Verbindungen der Formel I eignen sich z. B. optisch aktive Säuren, wie die D- und L-Formen von Weinsäure, Dibenzoylweinsäure, Diacetylweinsäure, Camphersulfonsäuren, Mandelsäure, Äpfelsäure oder Milchsäure. Carbinole (I, $R^3$ = OH) können ferner mit Hilfe chiraler Acylierungsreagenzien, z. B. D- oder L-α-Methylbenzylisocyanat, verestert und dann getrennt werden (vgl. EP-A1-120428). Die verschiedenen Formen der Diastereomere können in an sich bekannter Weise, z. B. durch fraktionierte Kristallisation, getrennt, und die Enantiomeren der Formel I können in an sich bekannter Weise aus den Diastereomeren in Freiheit gesetzt werden. Enantiomerentrennungen gelingen ferner durch Chromatographie an optisch-aktiven Trägermaterialien.

Die Verbindungen der Formel I und ihre physiologisch unbedenklichen Salze können zur Herstellung pharmazeutischer Zubereitungen verwendet werden, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human-oder Veterinärmedizin verwendet werden. Als Trägerstoffe kommen organische oder anorganische Substanzen in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z. B. zur Herstellung von Injektionspräparten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb- und Geschmacks- und/oder Aromastoffe enthalten. Sie können, falls erwünscht, auch einen oder mehrere weitere Wirkstoffe enthalten, z. B. ein oder mehrere

Vitamine.

Die Verbindungen der Formel I und ihre physiologisch unbedenklichen Salze können an Menschen oder Tiere, insbesondere Säugetiere wie Affen, Hunde, Katzen, Ratten oder Mäuse verabreicht und bei der therapeutischen Behandlung des menschlichen oder tierischen Körpers sowie bei der Bekämpfung von Krankheiten verwendet werden, insbesondere bei der Therapie und/oder Prophylaxe von Störungen des cardiovasculären Systems, insbesondere dekompensierter Herzinsuffizienz, Angina pectoris, peripheren oder cerebralen Gefäßerkrankungen, sowie Krankheitszuständen, die mit Bluthochdruck verbunden sind, ferner von Erkrankungen, die mit Veränderungen der nicht-vaskulären Muskulatur verbunden sind, z.B. Asthma, Inkontinenz der Harnblase.

Dabei werden die erfindungsgemäßen Substanzen in der Regel in Analogie zu bekannten Antianginosa bwz. Blutdrucksenkern, z. B. Nicorandil oder Cromakalim verabreicht, vorzugsweise in Dosierungen zwischen etwa 0,01 und 5 mg, insbesondere zwischen 0,02 und 0,5 mg pro Dosierungseinheit. Die tägliche Dosierung liegt vorzugsweise zwischen etwa 0,0001 und 0,1, insbesondere zwischen 0,0003 und 0,01 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachfolgenden Beispielen bedeutet "übliche Aufarbeitung":

Man gibt, falls erforderlich, Wasser hinzu, extrahiert mit einem organischen Lösungsmittel wie Ethylacetat, trennt ab, trocknet die organische Phase über Natriumsulfat, filtriert, dampft ein und reinigt durch Chromatographie und/oder Kristallisation.

Vor- und nachstehend sind alle Temperaturen in ° C angegeben.

Beispiel 1

Man löst 2,1 g 1,3-Cyclopentandion unter $N_2$ in 200 ml THF, fügt 650 mg NaH (80 % in Mineralöl) hinzu, rührt 1 Std., fugt nacheinander eine Lösung von 4,6 g 2,2,3-Trimethyl-3,4-epoxy-6-cyan-chroman ("IIa") in 20 ml THF, dann 2,4 ml $BF_3$-Etherat hinzu und rührt über Nacht bei 20°. Man dampft ein und reinigt das erhaltene 2,2,3-Trimethyl-4-(3-oxo-1-cyclopenten-1-yloxy)-6-cyan-3-chromanol ("A") chromatographisch an Kieselgel (Ethylacetat/Methanol 100:1); F. 204-206° (aus Isopropanol).

Analog erhält man mit 1,3-Cyclohexandion das 2,2,3-Trimethyl-4-(3-oxo-1-cyclohexen-1-yl-oxy)-6-cyan-3-chromanol.

Analog erhält man aus
25 2,2,3-Trimethyl-3,4-epoxy-chroman
2,2,3-Trimethyl-3,4-epoxy-6-chlor-chroman
2,2,3-Trimethyl-3,4-epoxy-6-brom-chroman
2,2,3-Trimethyl-3,4-epoxy-6-acetyl-chroman
2,2,3-Trimethyl-3,4-epoxy-6-methoxycarbonyl-chroman
2,2,3-Trimethyl-3,4-epoxy-6-ethoxycarbonyl-chroman
2,2,3-Trimethyl-3,4-epoxy-6-nitro-chroman
2,2,3-Trimethyl-3,4-epoxy-6-acetamido-chroman
2,2,3-Trimethyl-3,4-epoxy-7-acetamido-chroman
2,2,3-Trimethyl-3,4-epoxy-6-acetamido-7-nitro-chroman
2,2-Diethyl-3-methyl-3,4-epoxy-6-cyan-chroman
2,2-Tetramethylen-3-methyl-3,4-epoxy-6-cyan-chroman
2,2-Pentamethylen-3-methyl-3,4-epoxy-6-cyan-chroman mit 1,3-Cyclopentandion:
2,2,3-Trimethyl-4-(3-oxo-1-cyclopenten-1-yl-oxy)-3-chromanol
2,2,3-Trimethyl-4-(3-oxo-1-cyclopenten-1-yl-oxy)-6-chlor-3-chromanol
2,2,3-Trimethyl-4-(3-oxo-1-cyclopenten-1-yl-oxy)-6-brom-3-chromanol
2,2,3-Trimethyl-4-(3-oxo-1-cyclopenten-1-yl-oxy)-6-acetyl-3-chromanol
2,2,3-Trimethyl-4-(3-oxo-1-cyclopenten-1-yl-oxy)-6-methoxycarbonyl-3-chromanol
2,2,3-Trimethyl-4-(3-oxo-1-cyclopenten-1-yl-oxy)-6-ethoxycarbonyl-3-chromanol
2,2,3-Trimethyl-4-(3-oxo-1-cyclopenten-1-yl-oxy)-6-nitro-3-chromanol
2,2,3-Trimethyl-4-(3-oxo-1-cyclopenten-1-yl-oxy)-6-acetamido-3-chromanol
2,2,3-Trimethyl-4-(3-oxo-1-cyclopenten-1-yl-oxy)-7-acetamido-3-chromanol
2,2,3-Trimethyl-4-(3-oxo-1-cyclopenten-1-yl-oxy)-6-acetamido-7-nitro-3-chromanol

2,2-Diethyl-3-methyl-(3-oxo-1-cyclopenten-1-yl-oxy)-6-cyan-3-chromanol
2,2-Tetramethylen-3-methyl-(3-oxo-1-cyclopenten-1-yl-oxy)-6-cyan-3-chromanol
2,2-Pentamethylen-3-methyl-(3-oxo-1-cyclopenten-1-yl-oxy)-6-cyan-3-chromanol

Beispiel 2

Analog Beispiel 1 erhält man aus (-)-(3S,4S)-2,2,3-Trimethyl-3,4-epoxy-6-cyan-chroman ["(-)-IIa"] und 1,3-Cyclopentandion das (3S,4R)-2,2,3-Trimethyl-4-(3-oxo-1-cyclopenten-1-yl-oxy)-6-cyan-3-chromanol.

Analog erhält man aus (+)-(3R,4R)-2,2,3-Trimethyl-3,4-epoxy-6-cyan-chroman das (3R,4S)-2,2,3-Trimethyl-4-(3-oxo-1-cyclopenten-1-yl-oxy)-6-cyan-3-chromanol.

Beispiel 3

Man löst 2,15 g IIa in 30 ml DMSO unter $N_2$, gibt unter Rühren zunächst 0,2 g NaH, dann portionsweise 1,5 g 3-Amino-2-cyclopenten-1-on hinzu und rührt 24 Stunden bei 20°. Nach üblicher Aufarbeitung erhält man 2,2,3-Trimethyl-4-(3-oxo-1-cyclopenten-1-yl-amino)-6-cyan-3-chromanol ("B").

Analog erhält man aus IIa und 4-Amino-2(5H)-furanon das 2,2,3-Trimethyl-4-[2-oxofuran-4(5H)-yl-amino]-6-cyan-3-chromanol.

Analog erhält man aus IIa und 3-Methylamino-2-cyclopenten-1-on das 2,2,3-Trimethyl-4-[N-methyl-N-(3-oxo-1-cyclopenten-1-yl)-amino]-6-cyan-3-chromanol.

Analog erhält man aus IIa und 4-Methylamino-2(5H)-furanon das 2,2,3-Trimethyl-4-[N-methyl-N-(2-oxofuran-4(5H)-yl)-amino]-6-cyan-3-chromanol.

Analog erhält man aus IIa und 1-Methyl-4-methylamino-2(5H)-pyrrolon das 2,2,3-Trimethyl-4-[N-methyl-N-(1-methyl-2-oxo-pyrrol-4(5H)-yl)-amino]-6-cyan-3-chromanol.

Analog erhält man aus den entsprechenden 3,4-Epoxychromanen:
2,2,3-Trimethyl-4-(3-oxo-1-cyclopenten-1-yl-amino)-3-chromanol
2,2,3-Trimethyl-4-(3-oxo-1-cyclopenten-1-yl-amino)-6-chlor-3-chromanol
2,2,3-Trimethyl-4-(3-oxo-1-cyclopenten-1-yl-amino)-6-brom-3-chromanol
2,2,3-Trimethyl-4-(3-oxo-1-cyclopenten-1-yl-amino)-6-methoxycarbonyl-3-chromanol
2,2,3-Trimethyl-4-(3-oxo-1-cyclopenten-1-yl-amino)-6-ethoxycarbonyl-3-chromanol 2,2,3-Trimethyl-4-(3-oxo-1-cyclopenten-1-yl-amino)-6-nitro-3-chromanol.

Beispiel 4

Analog Beispiel 1 erhält man aus IIa und Tetronsäure das 2,2,3-Trimethyl-4-[2-oxofuran-4(5H)-yloxy]-6-cyan-3-chromanol.

Analog erhält man aus IIa und Thiotetronsäure das 2,2,3-Trimethyl-4-[2-oxothiophen-4(5H)-yloxy]-6-cyan-3-chromanol.

Beispiel 5

Ein Gemisch von 3,12 g "B", 20 g Ameisensäure-essigsäureanhydrid und 50 ml THF wird 7 Std. unter $N_2$ gerührt. Man dampft ein, arbeitet wie üblich auf und erhält 2,2,3-Trimethyl-4-[N-formyl-N-(3-oxo-1-cyclopenten-1-yl)-amino]-6-cyan-3-chromanol.

Beispiel 6

Eine Lösung von 3,12 g "B" in 150 ml Aceton wird dreimal in jeder Stunde mit 2,5 ml 37%iger wässeriger Formaldehydlösung gemischt und insgesamt 5 Std. bei pH 8-9 (Zugabe von Natronlauge!) gerührt. Nach ublicher Aufarbeitung erhält man 2,2,3-Trimethyl-4-(2-hydroxymethyl-3-oxo-1-cyclopenten-1-yl-amino)-6-cyan-3-chromanol.

Beispiel 7

Man oxidiert 1,4 g des nach Beispiel 6 erhaltenen Produkts mit 2,2 g Collin's Reagenz in 250 ml Dichlormethan während 10 Minuten, arbeitet wie üblich auf (Chromatographie an Silicagel; Dichlormethan/Methanol 97:3) und erhält 2,2,3-Trimethyl-4-(2-formyl-3-oxo-1-cyclopenten-1-yl-amino)-6-cyan-3-chromanol.

Beispiel 8

Eine Losung von 1,1, g "B" in einem Gemisch von 35 ml Dichlormethan und 35 ml Dioxan wird mit 700 mg Xenondifluorid gemischt, 5 Std. bei 20° gerührt und in Wasser gegossen. Nach üblicher Aufarbeitung erhält man 2,2,3-Trimethyl-4-(2-fluor-3-oxo-1-cyclopenten-1-yl-amino)-6-cyan-3-chromanol.

Beispiel 9

Eine Lösung von 156 mg "B" in einem Gemisch von 5 ml Dichlormethan und 5 ml Dioxan wird mit 77 mg N-Chlorsuccinimid 1 Std. bei 20° gerührt und wie üblich aufgearbeitet. Man erhält 2,2,3-Trimethyl-4-(2-chlor-3-oxo-1-cyclopenten-1-yl-amino)-6-cyan-3-chromanol.

Analog erhält man mit N-Bromsuccinimid das 2,2,3-Trimethyl-4-(2-brom-3-oxo-1-cyclopenten-1-yl-amino)-6-cyan-3-chromanol.

Beispiel 10

Ein Gemisch von 2 g "A", 11,7 ml Ameisensäure und 3,3 ml Acetanhydrid wird 16 Std. bei 20° stehengelassen und anschließend 2 Std. auf 40-45° erwärmt. Nach Eindampfen und üblicher Aufarbeitung erhält man 2,2,3-Trimethyl-3-formyloxy-4-(3-oxo-1-cyclopenten-1-yl-oxy)-6-cyanchroman.

Beispiel 11

Ein Gemisch von 1 g "A" und 5 ml Acetanhydrid wird 1 Std. gekocht. Man kühlt ab, arbeitet wie üblich auf und erhält 2,2,3-Trimethyl-3-acetoxy-4-(3-oxo-1-cyclopenten-1-yl-oxy)-6-cyan-chroman.

Beispiel 12

Eine Lösung von 3,46 g 2,2,3-Trimethyl-4-(3-oxo-1-cyclopenten-1-yl-oxy)-6-methoxycarbonyl-3-chromanol in 100 ml 33%iger ethanolischer Dimethylaminlösung wird 10 Tage in einem Autoklaven auf 100° erhitzt. Man kühlt ab, arbeitet wie üblich auf und erhält 2,2,3-Trimethyl-4-(3-oxo-1-cyclopenten-1-yl-oxy)-6-dimethylaminocarbonyl-3-chromanol.

Beispiel 13

In eine siedende Lösung von 1 g "A" in 50 ml Methanol und 2 ml Wasser wird 14 Std. unter Rühren HCl eingeleitet. Man läßt erkalten, arbeitet wie üblich auf und erhält 2,2,3-Trimethyl-4-(3-oxo-1-cyclopenten-1-yl-oxy)-6-carboxy-3-chromanol.

Beispiel 14

Ein Gemisch von 3,13 g "A", 31 g $Na_3PO_4 \cdot 12\ H_2O$, 28 ml Pyridin, 28 ml Wasser, 67 ml Essigsäure und 25 g Raney-Ni (wasserfeucht) wird 3 Std. bei 20° gerührt. Nach Filtration arbeitet man wie üblich auf und erhält 2,2,3-Trimethyl-4-(3-oxo-1-cyclopenten-1-yl-oxy)-6-formyl-3-chromanol.

Beispiel 15

Man löst 3,13 g "A" in 45 ml tert.-Butanol und gibt unter Rühren 5 g gepulvertes KOH hinzu. Nach 1std. Kochen und üblicher Aufarbeitung erhält man 2,2,3-Trimethyl-4-(3-oxo-1-cyclopenten-1-yl-oxy)-6-carbamoyl-3-chromanol.

Beispiel 16

In eine Lösung von 3,13 g "A" in einem Gemisch von 20 ml Pyridin und 10 ml Triethylamin leitet man 5 Std. bei 20° $H_2S$ ein, dampft ein, arbeitet wie üblich auf und erhält 2,2,3-Trimethyl-4-(3-oxo-1-cyclopenten-1-yl-oxy)-6-thiocarbamoyl-3-chromanol.

Die nachstehenden Beispiele betreffen pharmazeutische Zubereitungen, die Verbindungen der Formel I oder ihre physiologisch unbedenklichen Salze enthalten:

Beispiel A Tabletten

Ein Gemisch von 0,2 kg "A", 136,3 kg Calciumhydrogenphosphat, 15 kg Maisstärke, 10 kg mikrokristalliner Cellulose, 5,5 kg unlöslichem Polyvinylpyrrolidon (PVP), 1,5 kg hochdispersem $SiO_2$ und 1,5 kg Magnesiumstearat wird in üblicher Weise zu Tabletten gepreßt. Jede 170 mg-Tablette enthält 0,2 mg Wirkstoff.

Beispiel B Dragees

Analog Beispiel A, jedoch ohne den Zusatz von PVP, werden Tabletten gepreßt, die anschließend in üblicher Weise mit einem Überzug aus Saccharose, Kartoffelstärke, Talk, Tragant und Farbstoff überzogen werden.

Beispiel C Kapseln

Man bereitet ein Granulat aus 10 g "A", 27,5 kg Lactose, 0,35 kg Hydroxypropylmethylcellulose und 0,7 kg Maisstärke, mischt dieses mit 0,15 kg hochdispersem $SiO_2$ und 0,3 kg Magnesiumstearat und füllt das Gemisch in üblicher Weise in Hartgelatinekapseln, so daß jede Kapsel 0,1 mg Wirkstoff enthält.

Beispiel D Lacktabletten

Aus 0,2 kg "A", 151,3 kg Lactose, 10 kg mikrokristalliner Cellulose, 5,5 kg PVP, 1,5 kg hochdispersem $SiO_2$ und 1,5 kg Magnesiumstearat werden Tablettenkerne (170 mg) gepreßt, die anschließend in üblicher Weise lackiert werden, so daß jede Lacktablette mit 3,922 mg eines Lackes überzogen ist, der aus 2,2 mg Hydroxypropylmethylcellulose, 0,53 mg Polyethylenglykol 400, 0,85 mg $TiO_2$, 0,122 mg $Fe_2O_3$ und 0,22 mg Silikonöl besteht.

Beispiel E Ampullen

Eine Lösung 10 g "A" in 70 l 1,2-Propandiol wird mit zweifach destilliertem Wasser auf 100 l aufgefullt, steril filtriert und in 1-ml-Ampullen abgefüllt, die dann steril verschlossen werden. Jede Ampulle enthält 0,1 mg Wirkstoff.

Analog sind Tabletten, Dragees, Kapseln, Lacktabletten oder Ampullen erhältlich, die einen oder mehrere der übrigen Wirkstoffe der Formel I und/oder ihrer physiologisch unbedenklichen Salze enthalten.

**Ansprüche**

1. Chromanderivate der Formel I

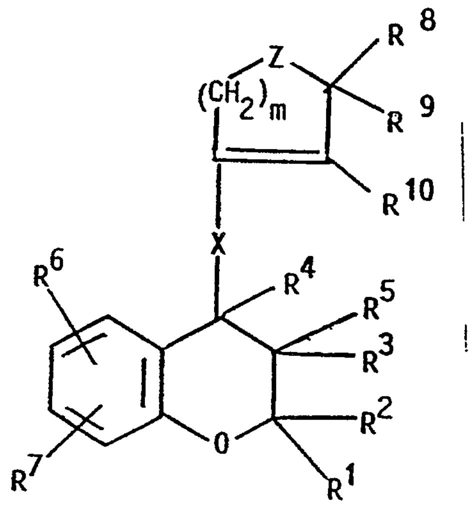

I

worin

X O oder $NR^{11}$,

Z $CH_2$, O, S oder CHHal,

$R^1$ und $R^5$ jeweils A,

$R^2$ H oder A,

$R^1$ und $R^2$ zusammen auch Alkylen mit 3-6 C-Atomen,

$R^3$ OH oder OAc,

$R^4$ H,

$R^3$ und $R^4$ zusammen auch eine Bindung,

$R^6$ und $R^7$ jeweils H, A, HO, AO, CHO, ACO, ACS, HOOC, AOOC, AO-CS, ACOO, A-CS-O, Hydroxyalkyl, $NO_2$, $NH_2$, NHA, $NA_2$, CN, F, Cl, Br, J, $CF_3$, ASO, $ASO_2$, AO-SO, AO-$SO_2$, AcNH, AO-CO-NH, $H_2NSO$, HANSO, $A_2NSO$, $H_2NSO_2$, $HANSO_2$, $A_2NSO_2$, $H_2NCO$, HANCO, $A_2NCO$, $H_2NCS$, HANCS, $A_2NCS$, ASONH, $ASO_2NH$, AOSONH, $AOSO_2NH$, ACO-alkyl, Nitro-alkyl, Cyan-alkyl, A-C(=NOH) oder A-C-(=$NNH_2$),

$R^8$ und $R^9$ jeweils H oder A oder zusammen =O oder =S,

$R^{10}$ H, Hal, CHO oder $CH_2OH$,

$R^{11}$ H, A, Ac oder $CH_2OH$,

m 1, 2 oder 3,

Hal F, Cl, Br oder J,

Ac Alkyl mit 1-6 C-Atomen,

alkyl Alkylen mit 1-6 C-Atomen und

A Alkanoyl mit 1-8 C-Atomen oder Aroyl mit 7-11 C-Atomen

bedeuten,

sowie deren Salze.

2. 2,2,3-Trimethyl-4-(3-oxo-1-cyclopenten-1-yl-oxy)-6-cyan-chroman-3-ol.

3. Verfahren zur Herstellung von Chromanderivaten der Formel I, dadurch gekennzeichent, daß man ein 3,4-Epoxychroman der Formel II

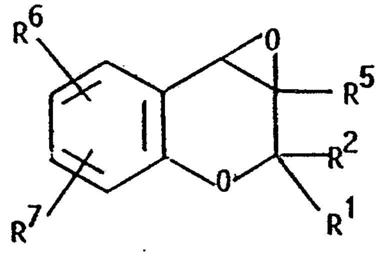

II

worin
$R^1$, $R^2$, $R^5$, $R^6$ und $R^7$ die bei Formel I angegebene Bedeutung haben,
mit einer Verbindung der Formel III

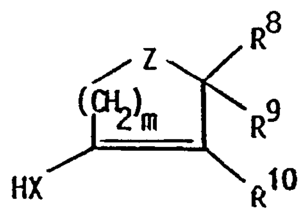

III

worin X, Z, $R^8$, $R^9$, $R^{10}$ und m die bei Formel I angegebene Bedeutung haben oder mit einem ihrer reaktionsfähigen Derivate umsetzt
und/oder daß man eine Verbindung der Formel I, worin $R^3$ OH und $R^4$ H bedeutet, dehydratisiert und/oder daß man in einer Verbindung der Formel I einen oder mehrere der Reste X, Z, $R^3$, $R^6$ und /oder $R^7$ in andere Reste X, Z, $R^3$, $R^6$ und/oder $R^7$ umwandelt und/oder daß man eine basische Verbindung der Formel I durch Behandeln mit einer Säure in eines ihrer Säureadditionssalze umwandelt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verbindungen der Formel I zur Bekämpfung von Krankheiten.

7. Verwendung von Verbindungen der Formel I zur Herstellung eines Arzneimittels.

8. Verwendung von Verbindungen der Formel I bei der Bekämpfung von Krankheiten.